(19)

**Europäisches Patentamt**
**European Patent Office**
**Office européen des brevets**

(11) **EP 4 201 326 A1**

(12) **EUROPEAN PATENT APPLICATION**

(43) Date of publication:
**28.06.2023 Bulletin 2023/26**

(21) Application number: **21216887.6**

(22) Date of filing: **22.12.2021**

(51) International Patent Classification (IPC):
**A61B 5/145** *(2006.01)*    **A61B 5/01** *(2006.01)*
**A61B 5/00** *(2006.01)*

(52) Cooperative Patent Classification (CPC):
**A61B 5/14532; A61B 5/01; A61B 5/14503;**
**A61B 5/1451; A61B 5/6813; A61B 5/6833;**
**A61B 5/742; A61B 5/746**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
• **F. Hoffmann-La Roche AG**
**4070 Basel (CH)**
Designated Contracting States:
**AL AT BE BG CH CY CZ DK EE ES FI FR GB GR**
**HR HU IE IS IT LI LT LU LV MC MK MT NL NO PL**
**PT RO RS SE SI SK SM TR**
• **Roche Diabetes Care GmbH**
**68305 Mannheim (DE)**
Designated Contracting States:
**DE**

(72) Inventors:
• **BAUMANN, Edgar**
**68305 Mannheim (DE)**
• **THOES, Bruno**
**68305 Mannheim (DE)**
• **WETTENGEL, Klaus**
**68305 Mannheim (DE)**

(74) Representative: **Freiherr von Campenhausen,**
**Harald**
**Roche Diagnostics GmbH**
**Patentabteilung**
**Sandhofer Straße 116**
**68305 Mannheim (DE)**

(54) **BODY WEARABLE ANALYTE SENSOR SYSTEM WITH NONCONTACT TEMPERATURE SENSOR**

(57)    The present invention provides analyte sensor system which comprises a body wearable analyte sensor device, comprising a transcutaneous analyte sensor, a housing comprising a lower side which can be attached to the skin of a patient, a noncontact temperature sensor which is adapted to detect the temperature of the lower side of the housing or to detect the temperature of the skin, and wherein the noncontact temperature sensor faces without contact the lower side of the housing, and a processor configured to receive signals from the analyte sensor and from the noncontact temperature sensor. In addition, a method of determing an analyte concentration using an analyte sensor system (1) of the invention is provided.

EP 4 201 326 A1

FIG. 2

1

11

2

410

7

91

8

4

33

331

3

6

5

41

412

**Description**

The present invention provides an analyte sensor system which comprises a body wearable analyte sensor device, comprising a transcutaneous analyte sensor, a housing comprising a lower side which can be attached to the skin of a patient, preferably with a plaster, a noncontact temperature sensor which is adapted to detect the temperature of the lower side of the housing or to detect the temperature of the skin, and wherein the noncontact temperature sensor faces without contact the lower side of the housing, and a processor configured to receive signals from the analyte sensor and from the noncontact temperature sensor. In addition, a method of determing an analyte concentration using an analyte sensor system of the invention is provided.

Monitoring certain body analytes plays an important role in the prevention and treatment of various diseases. Without restricting further possible applications, the invention will be described in the following text with reference to blood-glucose monitoring. However, the invention can also be applied to other types of analytes.

Blood glucose monitoring may be performed by using optical or electrochemical analyte sensors. Examples of electrochemical sensors for measuring glucose, specifically in blood or other body fluids, are known.

In addition to so-called spot measurements, in which a sample of a body fluid is isolated from a user in a targeted fashion and examined with respect to the analyte concentration, continuous determinations are increasingly becoming established. Thus, in the recent past, continuous measuring of glucose in the interstitial tissue (also referred to as continuous monitoring, CM) for example has been established as another important method for managing, monitoring and controlling a diabetes state.

In the process, the active sensor region of the analyte sensor system is applied directly to the detection site, which is generally arranged in the interstitial tissue, and, for example, converts glucose into electrical charge by using an enzyme (e.g. glucose oxidase, GOD), which charge is related to the glucose concentration and can be used as a detection variable.

Hence, hitherto known analyte sensor systems for continuous monitoring typically are transcutaneous systems or subcutaneous systems, wherein both expressions, in the following, will be used equivalently. This means that the actual sensor or at least a measuring portion of the sensor is arranged under the skin of the user. However, an evaluation and control part of the system is generally situated outside of the body of the user, outside of the human or animal body. In the process, the sensor is generally applied using an insertion instrument, which is likewise known in the art.

The sensor typically comprises a substrate, such as a flat substrate, onto which an electrically conductive pattern of electrodes, conductive traces and contact pads may be applied. In use, the conductive traces typically are isolated by using one or more electrically insulating materials. The electrically insulating material typically further also acts as a protection against humidity and other detrimental substances and, as an example, may comprise one or more cover layers such as resists.

As outlined above, in transcutaneous systems, a control part is typically required, which may be located outside the body tissue and which has to be in communication with the sensor. Typically, this communication is established by providing at least one electrical contact between the sensor and the control part, which may be a permanent electrical contact or a releasable electrical contact. Examples of electrical contacts for contacting a triangular assembly of contact pads are known in the art. Other techniques or providing electrical contacts, such as by appropriate spring contacts, are also generally known and may be applied.

In order to avoid detrimental effects of the aggressive environment onto the conductive properties of the electrical contact, the region of the electrical contact is typically encapsulated and protected against humidity. Generally, encapsulations of electrical locks and contacts by using appropriate seals is known.

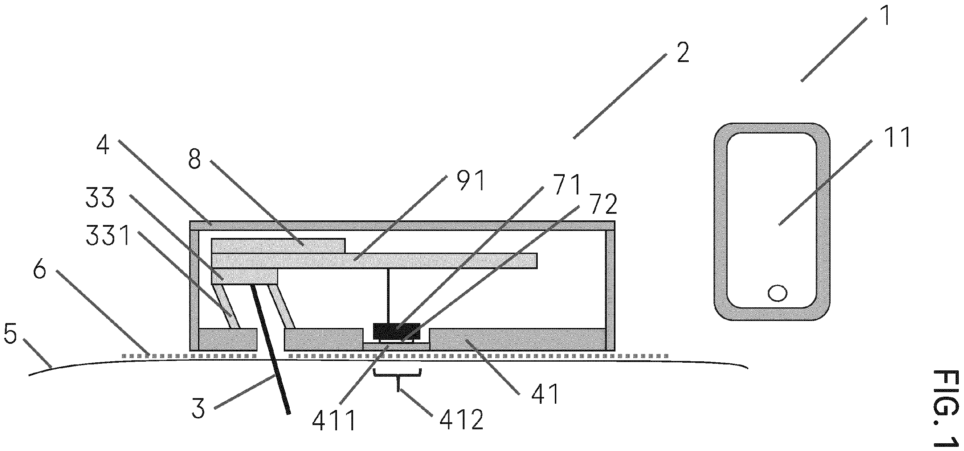

It is also generally known that the analyte detection of glucose analyte sensor and in particular the detection of the analyte concentration may be influenced by the temperature of the sensor. As a consequence, in order to achieve consistent determination of, e.g. the analyte concentration at varying temperatures of the body wearable analyte sensor device attached to the skin of the patient during use, the body wearable analyte sensor device is often equipped with a contact temperature sensor positioned in contact with a circuit board inside the housing of the body wearable analyte sensor device or in contact with the side of this housing attached to the skin of the patient (e.g. see Fig.1) and the contact temperature signals are included as a factor in addition to the signals from the analyte sensor in the determination of the analyte value such as the analyte concentration.

US20210259586A1 discloses a non-invasive optical glucose sensor system comprising a housing attached to the skin, a total reflection member configured to totally reflect an incoming probe beam in a state being in contact with a detected object, a glucose sensor, a temperature sensor which is essentially a sensor in contact with the object to be detected, a processor, and a temperature adjuster configured to maintain, to a predetermined temperature, a temperature of a contact region of the total reflection member with the detected object (e.g. skin) based on the temperature detection of the temperature sensor. In other words, the temperatures is detected in order to adjust the temperature of the contact region of the total reflection member but the temperature mearsurement is not used to determine a sensed analyte value.

Despite the advantages and the progress achieved by the above-mentioned developments, specifically in the field of continuous monitoring technology, some significant technical challenges remain. For example, the attachment of the contact temperature sensor to the housing of the body wearable analyte sensor device is associated with complex design and high production costs, e.g. because it is associated with additional wiring between the circuit board and the contact sensor. Moreover, if the contact sensor is attached to the circuit board heating up of the board may adversely affect the temperature detection by the contact temperature sensor that is intended to approximate the temperature of the skin essentially without influence from the temperature of the circuit board.

## Problem to be solved

It is therefore an objective of the present invention to provide an analyte sensor system and a method of determing an analyte concentration using an analyte sensor system, which at least partially avoid the shortcomings described above.

## Summary of the invention

At least one of the above objects is solved by the provision of the analyte sensor systems and the method of determing an analyte concentration using an analyte sensor system of the invention specified in the claims and herein below. Further advantages, features and technical effects of the present invention are apparent from the following description of embodiments and the accompanying figures.

According to a first aspect it is provided an analyte sensor system which comprises a body wearable analyte sensor device, comprising a transcutaneous analyte sensor, a housing) comprising a lower side which can be attached to the skin of a patient, preferably with a plaster, a noncontact temperature sensor which is adapted to detect the temperature of the lower side of the housing, or to detect the temperature of the skin, and wherein the noncontact temperature sensor faces without contact the lower side of the housing, and an electronic unit comprising a processor configured to receive signals from the analyte sensor and from the noncontact temperature sensor.

This invention is associated with the surprising effect that the impact of the skin temperature on the calculation of the analyte value can be assessed and corrected for more accurately compared to hitherto known systems because the detection of the noncontact temperatur sensor directly detects the temperature of the skin or of the side of the housing attached to the skin without being significantly impacted by the temperature of the noncontact temperature sensor itself. This effect therefore also provides for more flexibility in the technical design of the analyte system because the noncontact temperature sensor does not need to be positioned on the housing side attached to the skin but can also be positioned on an other part inside the housing, such as on a circuit board, where the noncontact temperature sensor faces the side of the housing attached to the skin. This way also glueing the temperature sensor to the housing side attached to the skin and the complex wiring of the temperature sensor with the circuit board can be avoided that is used in contact sensors based analyte sensor system (see e.g. Fig.1). Also in view of the above this invention is associated with the surprising effect that the production costs and time can be lowered compared to hitherto known systems.

Within the present invention the term "**analyte**" shall mean an arbitrary element, component or compound which may be present in the body fluid and the presence and/or the concentration of which may be of interest for the user, the patient or medical staff such as a medical doctor. Preferably, the analyte may be or may comprise an arbitrary chemical substance or chemical compound which may take part in the metabolism of the user or the patient, such as at least one metabolite. As an example, the at least one analyte may be selected from the group consisting of glucose, cholesterol, triglycerides, lactate. Additionally or alternatively, however, other types of analytes may be used and/or any combination of analytes may be determined. The detection of the at least one analyte specifically may be an analyte-specific detection. Preferably, the analyte is glucose.

Within the present invention the term "**sensor**" shall mean an arbitrary element which is adapted to perform a process of the detection and/or which is adapted to be used in the above-mentioned process of the detection. Thus, the sensor specifically may be adapted to determine the concentration of the analyte and/or a presence of the analyte. The sensor may particularly be a "transcutaneous analyte sensor".

As used herein, the term "**transcutaneous analyte sensor**" shall mean a sensor which is adapted to detect an analyte and to be fully or at least partly arranged within the interstitial fluid of skin or of under the skin of the patient. For this purpose, the analyte sensor generally maybe dimensioned such that a transcutaneous insertion is feasible, such as by providing a width in a direction perpendicular to an insertion direction of no more than 5 mm, preferably of no more than 2 mm, more preferably of no more than 1.5 mm. The sensor may have a length of less than 50 mm, such as a length of 30 mm or less, e.g. a length of 5 mm to 30 mm. It shall be noted, however, that other dimensions are feasible. In order to further render the sensor to be usable as a transcutaneous sensor, the sensor may fully or partially provide a biocompatible surface, i.e. a surface which, at least during durations of use, do not have any detrimental effects on the user, the patient or the body tissue. As an example, the transcutaneous sensor may fully or partially be covered with at least one biocompatible membrane, such as at least one polymer membrane or gel membrane which is permeable for the at least one analyte and/or the at least one body fluid and which, on the other hand, retains sensor substances such as one or more test chemicals within the sensor and prevents a migration of these substances into the body tissue. The sensor preferably may be an electrochemical analyte sensor. As used herein, an electrochemical analyte sensor generally is a sensor which is configured to conduct an electrochemical detection in order to detect the at least one analyte contained in the body fluid, preferably in the insterstitial fluid of the skin. The term "electrochemical detection" refers to a detection of an electrochemically detectable property of the analyte, such as an electrochemical detection reaction. Thus, for example, the electrochemical detection reaction may be detected by comparing one or more electrode potentials, as further discussed below. The electrochemical sensor specifically may be adapted to and/or may be usable to generate at least one electrical sensor signal which directly or indirectly indicates the presence and/or the extent of the electrochemical detection reaction, such as at least one current and/or at least one voltage. For this purpose, as will be outlined in further detail below, the at least one electrochemical analyte sensor provides two or more electrodes, which also are referred to as a sensor electrodes. The detection maybe analyte-specific. The detection maybe a qualitative and/or a quantitative detection.

Within the present invention the term "**analyte sensor system**" shall mean a kit of parts that comprises at least a body wearable analyte sensor device and an an electronic unit. Additional components which may be part of the analyte sensor system include at least one of (1) an insertion device for inserting the analyte sensor into the skin, (2) a cradle adapted to mechanically attach to the skin with its proximal side via a plaster bonded to the cradle and to the body wearable analyte sensor with its distal site, (3) at least one accessory such as a plaster, a cover, a package, a battery charger, an electronic consumer device adepted to communicate wirelessly or in a wired fashion with and/or in electrical connection with the body wearable analyte sensor device and/or with the electronic unit, the electronic consumer device maybe a smartphone, a pager, a smartwatch or any other electronic wearable device.

Within the present invention the term "**body wearable analyte sensor device**" shall mean that the analyte sensor device can be mounted on the body of the patient, preferably on the skin on any body part of the patient such as on the belly, an arm, a leg, the hip, the abdomen, the neck, an ear, the face, etc.

Within the present invention the term "**analyte sensor device**" shall mean a group of at least two elements which may interact in order to fulfill at least one common function. The at least two components may be handled independently or may be coupled, connectable or integratable in order to form a common component. Thus, an "**analyte sensor device**" generally refers to a group of at least two elements or components which are capable of interacting in order to perform at least one analyte sensor function, in the present case in order to perform at least one detection of an analyte in the body fluid and/or in order to contribute to the at least one detection of an analyte in the body fluid. The analyte sensor device may particularly be a device wherein the sensor is wholly or at least partly arranged within the body tissue of the patient. At least one component of the sensor system may be wholly or partly outside of the body tissue, for example the housing. During use the distal sensing part of the analyte sensor protrudes from the housing and is positioned in the skin or under the skin of the patient whereas the proximal non-sensing part of the analyte sensor connects the sensing part with the interior of the housing, specifically the distal part is electrically connected to the circuit board of the body wearable analye sensor device.

Within the present invention the term "**housing**" shall mean an arbitrary element which is adapted to surround one or more elements in order to provide one or more of a mechanical connection protection, an environmental protection against moisture and/or ambient atmosphere, a shielding against electromagnetic influences or the like. Specifically, the housing may be configured to shield one or more elements of the body wearable analyte sensor device from external influences like moisture and/or mechanical stress. The housing may be a watertight housing having an essentially round shape. Further, the housing may have an essentially flat lower side facing the skin. The housing, in general, may comprise one or more parts. The housing can be made of any material conventionally used to manufacture body worn medical devices such as from plastic or metal, preferably from plastic. Examples of suitable plastics are polycarbonate and polyethylen HD.

Within the present invention the term "**lower side of the housing**" shall mean the side of the housing attached to the skin either directly with a plaster or indirectly via a cradle where the housing can be mounted on. Preferably the side is directly attached to the skin with a plaster. The lower side is intended to mean the wall of the housing amongst the housing walls that is attached to the skin and/or which is positioned closest to the skin.

Within the present invention the term "**patient**" shall mean a human being or an animal, independent from the fact that the human being or animal, respectively, may be in a healthy condition or may suffer from one or more diseases. As an example, the patient or the user may be a human being or an animal suffering from diabetes. However, additionally or alternatively, the invention may be applied to other types of users or patients or diseases.

Within the present invention the term "**plaster**" shall mean an attachment component which is capable of connecting the body wearable analyte sensor device to the skin. The plaster comprises at least one adhesive surface and/or at least one adhesive strip that can be attached to the skin. The opposite side of the plaster can be attached to the lower side of the housing or to a cradle with an adhesive surface, though this attachment can also be a welded / covalent connection.

Within the present invention the term "**detect the temperature of the lower side of the housing**" shall mean that the temperature sensor detects, ascertains and/or quantifies the temperature of the lower side of the housing. In particular, the temperature sensor detects the temperature of the internal surface of the lower side of the housing facing the sensor, preferably in the temperature detection area of the lower side of housing. It is clear to the skilled person that such detected temperature will provide a reliable approximation of the temperature of the skin given that the skin temperature will cause the attached lower side of the housing to adapt to the skin temperature. The accuracy of that approximation increases as the thickness of the lower side or at least the thickness in the detection area of the lower side is reduced and/or the heat conductance properties of the material used for the lower side of the housing increases.

Within the present invention the term "**detect the temperature of the skin**" shall mean that the temperature sensor detects, ascertains and/or quantifies the temperature of skin which is preferably not covered by any lower side and preferably also not by any other part such as a plaster or cradle so that the noncontact sensor can direcly detect the temperature of the unobstructed skin, preferably in the detection area is located under a hole in the lower side of the housing which exposes the skin to direct and unobstructed detection by the noncontact temperature sensor.

Within the present invention the term "**noncontact temperature sensor faces without contact the lower side of the housing**" shall mean that essentially no physical object is located between the noncontact temperature sensor and the lower side of the housing. Preferably, the term means that no physical object is located between the temperature representing sensor stimulus (e.g. IR radiation emitted from the detected lower side) detecting part of the noncontact temperature sensor and the lower side of the housing, preferably the measuring area of the lower side of the housing, preferably the measuring area of the inner surface of the lower side of the housing.

In a preferred embodiment the housing of the body wearable analyte sensor device comprises an electronic unit which comprises a processor configured to receive signals from the analyte sensor and from the noncontact temperature sensor. The electronic unit is adapted to controlling the detection of the analyte or for transmitting sensor analyte data to another component such as a display device configured to communicate with the body wearable analyte sensor device and/or to another electronic device that lacks a display. The electronic unit comprises at least two electrical contacts which are electrically connected to the contact pads of the sensor. The electronic unit also comprises the circuit board, preferably the printed circuit board, of the invention.

Within the present invention the term "**electronic unit**" shall mean a device having at least one electronic component. Specifically, the electronic unit may comprise at least one electronic component for one or more of processing of analyte sensor signals, sensor, performing a voltage detection, performing a current detection, recording sensor signals, storing detection signals or detection data, transmitting sensor signals or detection data to another device. Other embodiments of the electronic components are feasible. The electronic unit specifically may comprise at least one circuit board having disposed thereon at least one electronics component, such as at least one active and/or at least one passive component. The electronic unit may further comprise at least one housing which fully or partially surrounds the electronics component. The electronic unit may further comprise at least one of an integrated circuit, a microcontroller, a computer or an application-specific integrated circuit (ASIC). The electronic unit may specifically be embodied as a transmitter or may comprise a transmitter, for transmitting data. Preferably, the electronic unit may be reversibly connectable to the body mount.

According to another embodiment the electronic unit is part of a transmitter unit component which is a component separte from the wearable analyte sensor device but which transmitter unit can be mechanically and electrically coupled to the wearable analyte sensor device. In this embodiment the circuit board is not comprised by the electronic unit but by the body wearable analyte sensor device. The electronic unit is adapted to receive analyte sensor data from the processor of the circuit board both comprised by the body wearable analyte sensor device. The electronic unit is also adapted to communicate the received analyte sensor data to a display device.

In another embodiment of the analyte sensor system of the invention the noncontact temperature sensor is mounted on a circuit board, preferably on a printed circuit board.

In another embodiment of the analyte sensor system of the invention the noncontact temperature sensor is a noncontact infrared sensor.

Within the present invention the term "**noncontact temperature sensor**" shall mean a temperature sensor that detects the temperature of an object by detecting the infrared radition emitted by the object. Such noncontact infrared sensors are for example available from Melexis Technologies NV, Belgium such as the MLX90632 noncontact infrared temperature sensor which is factory calibrated and used for body temperature detection and determination.

In another embodiment of the analyte sensor system of the invention the housing further comprises a contact temperature sensor.

Within the present invention the term "**contact temperature sensor**" shall mean a sensor that detects the temperature of its environment such as the object where it is mounted on. Examples of contact temperature sensors are generally known to the skilled person and may be selected from the group consisting of a thermoelement, a thermistor, and a resistance temperature detector.

In another embodiment of the analyte sensor system of the invention the contact temperature sensor is attached to a part of the housing carrying the noncontact temperature sensor or to the circuit board. Within the present invention the term "**attached to a part of the housing**" shall mean any form of mechanical, chemical connection between the contact temperature sensor and the part it is mounted on including a housing side, a circuit board, etc. The closer the distance is between the contact temperature sensor and the noncontact temperature sensor the more accurate the contact sensor can detect the temperature of the part the noncontact temperature sensor is attached to or the temperature of the noncontact temperature sensor itself. In a preferred embodiment this contact sensor based temperature detection is taken into account or checked when determining the analyte concentration based on the analyte sensor detection signal and the temperature signal by the noncontact temperature sensor. This way any adverse effect on the accuracy of the noncontact temperature sensor's temperature detection caused by an inappropriately high or low temperature of the noncontact temperature sensor itself or of the part or component it is attached to can be uncovered and corrected for in the determining of the analyte concentration.

In another embodiment of the analyte sensor system of the invention the wherein the lower side of the housing comprises a hole defining a temperature detection area of the skin configured such that the temperature of the skin below the hole can be detected by the noncontact temperature sensor. The direct detection of the skin temperature that is not obscured by a housing wall is associated with the surprising advantage that the skin temperature can be determined more accurately and temporal inertia effect of the housing wall on the temperature detection is essentially absent, thus increasing the accuracy of dynamic temperature detection and determination over time. This in turn, improves the accuracy of analyte value determination.

Within the present invention the term "**hole defining** a **temperature detection area of the skin**" shall mean an opening in the lower side of the housing that exposes the skin underneath the hole to the unobstructed access by the noncontact temperature sensor. The size and shape of the hole can have any kind of shape that the skilled person can readily determine bearing in mind measurement requirements determined by the detection field, area or angle of the noncontact sensor, requirements for avoiding ingress of dirt, fluid or humidity, etc.. Preferably, the hole has a circular, elipsoid, square or rectangular shape and preferably a maximum diameter of 0.5 to 3 cm, 0.5 to 2 cm, 0.5 to 1 cm, or 1 to 2 cm.

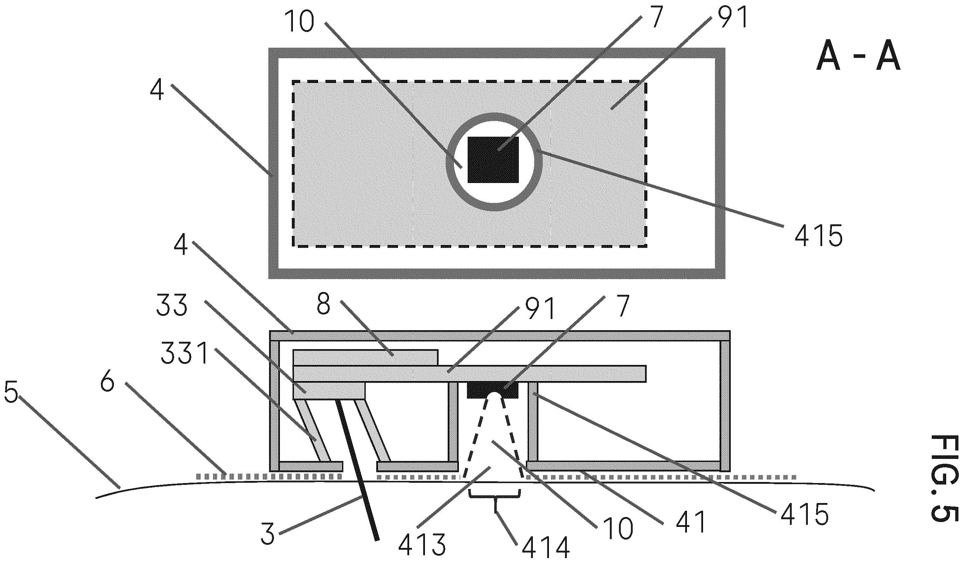

In another embodiment of the analyte sensor system of the invention a detection cell comprises the noncontact temperature sensor, the lower side of the housing comprising the hole, and separating means, preferably separating walls, sealing off the detection cell from the remaining interior space of the housing. Within the present invention the term "**detection cell**" shall mean the space between the noncontact temperature sensor, the lower side of the housing comprising the hole, and separating means. This is for example illustrated in Figure 5. Preferably, the detection cell provides a sealing off of the detection cell vis-à-vis remaining interior space of the housing against ingress from fluid, humidity, and particles.

Within the present invention the term "**separating means**" shall mean a wall, a seal or a membrane that protects aggainst ingress from fluid, humidity, and particles.

In another embodiment of the analyte sensor system of the invention the lower side of the housing in a temperature detection area detected by the noncontact temperature sensor has a reduced thickness relative to the thickness of other areas of the lower side. The temperature detection area of the lower side can be the same part as the rest of the lower side or it can be a separate part which is integrated or connected to the rest of the lower side. If it is a separate part, temperature detection area can be made of the same or of different material than the rest of the lower side.

The The thickness of the lower side of the housing ranges from 0.03-0.5 cm, preferably from 0.03-0.3 cm, preferably from 0.03-0.1 cm. Within the present invention the term "**reduced thickness relative to the thickness of other areas of the lower side**" shall mean that the reduced thickness ranges from ranges from 0.1-0.7 cm, preferably from 0.1-0.5 cm, preferably from 0.1-0.2 cm; preferably the thickness is reduced by 10 to 90%, preferable by 10 to 70%, preferable by 10 to 50%, preferable by 10 to 30%, relative to the thickness of other areas of the lower side.

The body wearable analyte sensor devices of the invention ranges in their maximum length of the housing from 0.5 to 5 cm, preferably from 0.5 to 3 cm, preferably from 0.5 to 2 cm. The maximum width of the housing ranges from 0.5 to 5 cm, preferably from 0.5 to 3 cm, preferably from 0.5 to 2 cm. The maximum length of the lower side of the housing ranges from 0.5 to 5 cm, preferably from 0.5 to 3 cm, preferably from 0.5 to 2 cm. The maximum width of the lower side of the housing ranges from 0.5 to 5 cm, preferably from 0.5 to 3 cm, preferably from 0.5 to 2 cm.

In another embodiment of the analyte sensor system of the invention the analyte system further comprises a display device configured to communicate with the body wearable analyte sensor device. Within the present invention the term "**display device**" shall mean any electronic device that comprises as display, preferably a touch display, such as a smartphone, a pager, and a wearable including a smartwatch. The display device is configured to communicate wiressly or in a wired fashion with the body wearable analyte sensor device or a part thereof, with the electronic unit and/or with the transmitter unit.

In another embodiment of the analyte sensor system of the invention the analyte sensor is a glucose sensor. Within the present invention the term "**glucose sensor**" shall mean an optical or electrochemical analyte sensor configured to detect glucose. Such electrochemical biosensors for measuring glucose, specifically in blood or other body fluids, are generally known. The sensor may comprise an enzyme such as glucose oxidase and/ or glucose dehydrogenase.

In another embodiment of the analyte sensor system of the invention the processor comprises a memory with instructions which when executed cause the processor to receiving a temperature detection signal from the noncontact temperature sensor, and determining the temperature of the lower side of the housing, preferably of the internal surface of the lower side, or of the temperature of the skin based on the received temperature detection signal from the noncontact temperature sensor, and optionally also based on a signal received from the contact temperature sensor.

Within the present invention the term **"receiving a temperature detection signal"** shall mean that a signal, preferably an electrical signal, from a temperature sensor representing at least one parameter of the detected temperature, such as a qualitative or quantitive temperature parameter, which signal is received by an electrical or electronic component, such as by a wire or a processor, preferably by a processor or a memory.

Within the present invention the term **"determining the temperature of the lower side of the housing"** shall mean a process of generating at least one representative result or a plurality of representative results indicating the temperature of the lower side of the housing. The temperature is preferably determined by the processor or by the electrical unit or a part thereof or by the circuit board or a part thereof. The temperature determination is based on at least one received noncontact temperature sensor signals from a noncontact temperature sensor and optionally also based on at least one contact temperature sensor signal from a contact temperature sensor.

Within the present invention the term **"internal surface of the lower side"** shall mean the surface of the lower side of the housing facing the noncontact temperature sensor. This is also the surface of the lower side that is oriented towards the interior of the housing, i.e. the surface of the lower side that is opposite of the other surface of the lower side which faces the plaster and the skin of the patient during use.

Within the present invention the term **"signal received from the contact temperature sensor"** shall mean a signal, preferably an electrical signal, from a contact temperature sensor representing at least one parameter of the detected temperature, such as a qualitative of quantitive temperature parameter, which signal is received by an electrical or electronic component, such as by a wire or a processor, preferably by a processor or a memory.

In another embodiment of the analyte sensor system of the invention the memory comprises further instructions which when executed cause the processor to receiving an analyte sensor detection signal from the transcutaneous analyte sensor, determining an analyte concentration based on the analyte sensor detection signal and the determined temperature. Preferably the determined temperature represents the temperature detected by the noncontact temperature. Alternatively, the determined temperature is the temperature detected by the noncontact temperature sensor and by the contact temperature sensor, and communicating the analyte concentration to the display device.

Within the present invention the term **"determining an analyte concentration"** shall mean a process of generating at least one representative result or a plurality of representative results indicating the concentration of the analyte in the body fluid. The analyte concentration is preferably determined by the processor or by the electrical unit or a part thereof or by the circuit board or a part thereof. The analyte concentration determination is based on at least one received analyte sensor detection signal from the transcutaneous analyte sensor and may preferably be further based on other parameters such as analyte sensor calibration data, at least one noncontact temperature sensor signal. Alternatively, the temperature sensor signal is additionally based on at least one contact temperature sensor signal.

Within the present invention the term **"communicating the analyte concentration to the display device"** shall mean the process of wired or wireless transmittion of a analyte concentration from the body wearable analyte sensor device, preferably from the processor to the display device, preferably via a transceiver comprised by the electrical unit and/or by the body wearable analyte sensor. The display device may then further process, display the analyte concentration on its display, and/or transmit the analyte concentration to another device via a network connection.

In another embodiment of the analyte sensor system of the invention the processor comprises further instructions which when executed cause the processor to comparing the determined temperature with a first and second predetermined reference temperature, and issueing a notification if the determined temperature is above the first predetermined reference temperature or below the second predetermined reference temperature, wherein the first predetermined reference temperature is higher than the second predetermined reference temperature. Preferably the determined analyte concentration was detected at a time when the determined temperature was detected to be above the first predetermined reference temperature or below the second predetermined reference temperature.

Preferably, the first predetermined reference temperature is between 40 to 50 °C, preferably between 40 to 45 °C , preferably between 40 to 42 °C . Preferably, the second predetermined reference temperature is between 0 to 15 °C , preferably between 5 to 15 °C , preferably between 10 to 15 °C , preferably between 5 to 10 °C . Preferably, the first predetermined reference temperature is between 40 to 45 °C, and the second predetermined reference temperature is between 5 to 10 °C.

Within the present invention the term **"predetermined reference temperature"** shall mean a reference temperature that is set and stored in the body wearable analyte sensor device or a part thereof, preferably in a memory. The predetermined reference temperature can be set and stored before hand over of the wearable analyte sensor device to the patient, such as during manufacturing or by a health care professional or by a patient before the wearable analyte sensor device is used on the body for the first time. Moreover, the predetermined reference temperature may also be adapted during use of the body wearable analyte sensor device.

This embodiment is associated with the surprising advantage that it improves the safety of analyte measurement: the patient is informed if the determined temperature of the lower side of the housing, or the skin, respectively, is such that there is a risk that determination of the analyte concentration with the transcutanous analyte sensor is inaccurate and/or unreliable because the determined temperature is too high (i.e. above the first predetermined reference temperature) or too low (i.e. below the second predetermined reference temperature). A person skilled in the art may readily determine such temperature ranges for the lower side of the housing and the skin, respectively, where accurate and/or reliable operation of the transcutaneous analyte sensor is at risk such as based on regulatory requirements defined by a regulatory body such as the TUV or Food and Drug Administration (FDA), or based on simulation or empirical testing of analyte concentration detection using an analyte sensor system of the invention in relation to determined temperatures in the skin (or a skin model system) exhibiting defined concentrations of the analyte of intered to be determined and also determined temperatures measured by the noncontact and contact temperature sensors of the invention. This in turn, will allow the skilled person to define adequate predetermined first and second reference temperatures.

Within the present invention the term **"issueing a notification"** shall mean that it is issued a message, alarm or signal that can be perceived by the patient. The notification preferably informs that the analyte concentration is at least one of the following: not reliable, cannot be trusted, cannot be used for treatment decisions such as decising on dosing or administration of a medicament, will not be stored, and will not be displayed.

In a preferred embodiment of the analyte sensor system of the invention the processor comprises further instructions which when executed cause the processor to comparing the determined temperature with a first and second predetermined reference temperature, and interrupting analyte detection by the transcutaneous analyte sensor, deleting or not storing data of the determined analyte concentration as long as the determined temperature is above the first predetermined reference temperature or below the second predetermined reference temperature, wherein the first predetermined reference temperature is higher than the second predetermined reference temperature. Preferably the determined analyte concentration was detected at a time when the determined temperature was detected to be above the first predetermined reference temperature or below the second predetermined reference temperature. Preferably, the interrupting analyte detection by the transcutaneous analyte sensor is interupted until the determined temperature is below the first predetermined temperature and above the second predetermined temperature.

In a preferred embodiment the determined temperature used for the termination of the analyte concentration is based on a signal received by the processor by the noncontact temperature sensor, while the determined temperature received by the processor for interrupting analyte detection by the transcutaneous analyte sensor, deleting or not storing data of the determined analyte concentration is detected by a contact sensor of the invention. In this embodiment the contact sensor functions as a safety device to reveal if the temperature of or around the noncontact temperature sensor is still within or outside the temperature range where it operates properly in order to ensure that adequate temperature values can be provided by the noncontact temperature sensor for determinining the analyte concentration. Thus, in this embodiment the noncontact sensor signals are not included in the determination of the analyte concentration if the temperature of or around the noncontact temperature sensor is outside the temperature range where it operates properly.

In another embodiment of the analyte sensor system of the invention a method of determing an analyte concentration using an analyte sensor system of the invention is provided, the method comprising the steps of: receiving a temperature detection signal from the noncontact temperature sensor, determining the temperature of the internal surface of the lower side of the housing or of the temperature of the skin a based on the received temperature detection signal from the noncontact temperature sensor, and optionally also based on a signal received from the contact temperature sensor, receiving an analyte sensor detection signal from the transcutaneous analyte sensor, determining an analyte concentration based on the analyte sensor detection signal and the determined temperature, and communicating the analyte concentration to the display device.

In another embodiment of the analyte sensor system of the invention a method of determing an analyte concentration using an analyte sensor system of the invention is provided, the method further comparing the determined temperature with a predetermined reference temperature, and communicating to the display device a notification if the determined temperature is above of below a predetermined reference temperature.

**Figure Legend**

The invention is further illustrated by the embodiments described and shown in the Figures and herein below.

Figure 1    shows a schematic frontal view of an analyte sensor system with contact temperature sensor known in the art.
Figure 2    shows a schematic frontal view of an analyte sensor system according to an embodiment of the present invention.

Figure 3    shows a schematic frontal view of an analyte sensor system according to another embodiment of the present invention.

Figure 4    shows a schematic frontal view of an analyte sensor system according to another embodiment of the present invention.

Figure 5    shows a schematic bottom-up view of an analyte sensor system according to the embodiment shown in Figure 4.

Figure 6    shows a temperature correction function described further in the Example, below.

**Detailed description of the invention**

The following Figures 1 to 5 are only schematic figures that do not limit the proportion or dimension of the depicted embodiments or elements thereof.

**Figure 1** shows a schematic frontal view of an analyte sensor system with contact temperature sensor known in the art which comprises an analyte sensor system (1) for continuous glucose determination which comprises a body wearable analyte sensor device (2) that communicates with a display device (11). The body wearable analyte sensor device (2) can be mounted on the skin and maintained in the mounted position by way of a plaster (6). The body wearable analyte sensor device (2) comprises a transcutaneous analyte sensor (3) which when inserted into the skin can detect glucose in the interstitial fluid of or under the skin (5). The transcutaneous analyte sensor (3) can be held by transcutaneous analyte sensor holder (33) which in addition to providing mechanical support for the sensor also electrically couples the transcutaneous analyte sensor's (3) electrode wires with the circuit board (91) which can be a printed circuit board that also comprises a processor (8) with a memory and a transceiver (not shown) for communicating with the display device (11). To prevent that humidity, liquid or dirt enters the interior of the housing separating walls (331), that are preferably waterproof, separate the space around the transcutaneous analyte sensor (3) between the transcutaneous analyte sensor holder, the separating walls (331) and the lower side of the housing around the hole where the sensor protrudes from the housing in transcutaneous placement position from the remainder of the internal housing. The circuit board (91) is comprised by the electronic unit (9, not shown) and both receive their power from a connected battery (not shown) that can be located within the housing (4). The circuit board (91) is in wired connection with a contact temperature sensor (71) which is attached by glue (72) to the interior surface of the lower side of housing (41) which side faces away from the skin (5) of the patient. The housing (4) and specifically the outer surface of its lower side (41), i.e. opposite of the inner surface, is attached to the skin (5) via a plaster (6). To reduce the temperature inertia effect of the lower side of the housing on the temperature detection by the contact temperature sensor (71) the thickness of the lower side (41, 411), preferably of the wall of the lower side, is reduced in the temperature detection area of the lower side of housing (412). During use, the analyte concentration is determined based on the analyte sensor signals received by the processor (8) from the transcutaneous analyte sensor (3) and may also be based on the temperature signals received by the processor (8) from the contact temperature sensor (71).

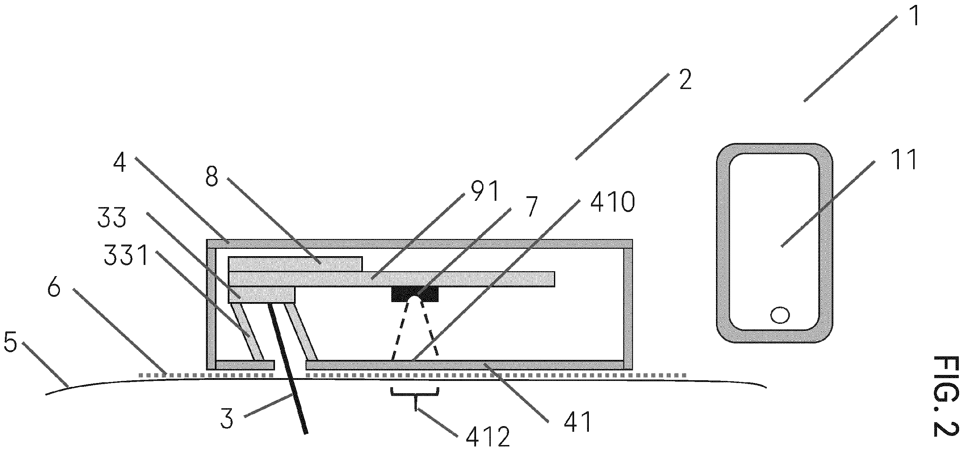

**Figure 2** shows a schematic frontal view of an analyte sensor system (1) according to an embodiment of the invention which comprises a body wearable analyte sensor device (2) and a display device (11) in communication with the body wearable analyte sensor device (2). The body wearable analyte sensor device (2), in turn, comprises a transcutaneous analyte sensor (3) which when inserted into the skin or under the skin can detect glucose in the interstitial fluid of the skin or under the skin. The transcutaneous analyte sensor (3) is held by transcutaneous analyte sensor holder (33) which in addition to providing mechanical support for the sensor also electrically couples the transcutaneous analyte sensor's (3) electrode wires with the circuit board (91, not shown) comprised by the electronic unit (9) which circuit board (91) can be a printed circuit board that also comprises a processor (8) with a memory (81, not shown) and a transceiver (not shown) for communicating with the display device (11). To prevent that humidity, liquid or dirt enters the interior of the housing separating walls (331), that are preferably waterproof, separate the space around the transcutaneous analyte sensor (3) between the transcutaneous analyte sensor holder, the separating walls (331) and the lower side of the housing around the hole where the sensor protrudes from the housing in transcutaneous placement position from the remainder of the internal housing. The circuit board (91) receives its power from a connected battery (not shown) that is also located within the housing (4). The circuit board (91) is in electrical connection with a noncontact temperature sensor (7) attached to the circuit board (91). The housing (4) and specifically the outer surface of its lower side (41) is attached to the skin via a plaster (6). The noncontact temperature sensor (7) is adapted to detect the temperature of the internal surface of the lower side of the housing (41) or to detect the temperature of the skin (5). The noncontact temperature sensor (7) faces without contact the lower side of the housing (41), preferably the internal surface of the lower side of housing opposite of the surface facing the skin (410) and detects the temperature in the temperature detection area of the lower side of housing (412). To reduce the temperature inertia effect of the lower side of the housing (41) on the temperature detection by the noncontact temperature sensor (7) the thickness of the lower side of the housing (41), particularly of the wall of the lower side of the housing (41), is reduced. During use, the analyte concentration is determined based on the analyte sensor signals received by the processor (8) from the transcutaneous analyte sensor (3) and based on the temperature signals received by the processor (8) from the noncontact temperature sensor (7).

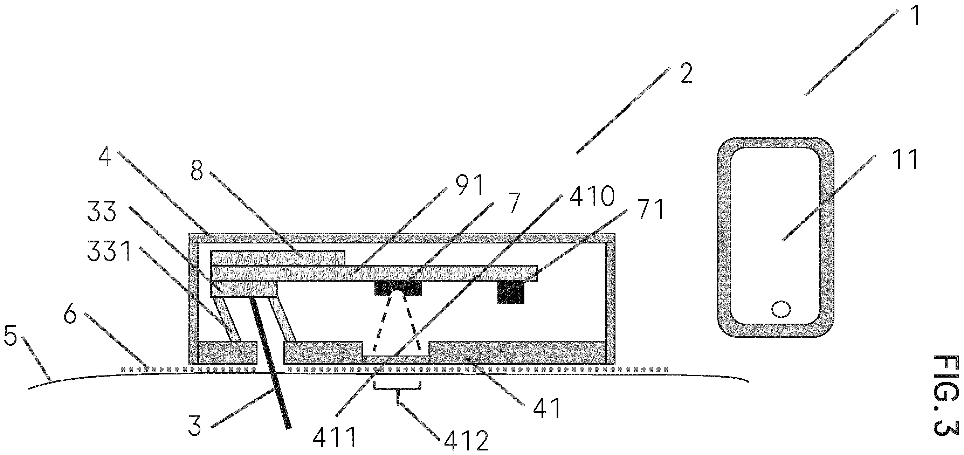

**Figure 3** shows a schematic frontal view of an analyte sensor system (1) according to another embodiment of the invention. This embodiment differs from the one described for Figure 2 only in the following aspects: firstly, the thickness of the lower side of the housing (41, 411), particularly of the the wall of the lower side of the housing (41, 411), is reduced only in the area of the internal surface of the lower side of housing opposite of the surface facing the skin (410), specifically in the temperature detection area of the lower side of housing (412). Secondly, the circuit board is additionally equipped with a contact temperature sensor (71). This way any impact of the temperature of the circuit board (9) can be taken into account that may adversely affect the temperature detection by the noncontact temperature sensor (7), if, e.g. the noncontact temperature sensor (7) is heated up by the circuit board (9) beyond its approved temperature operating range.

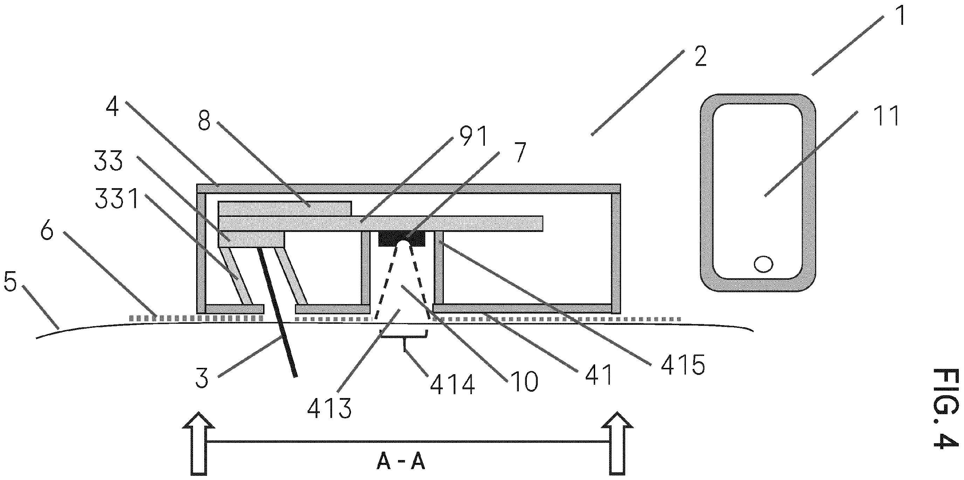

**Figure 4** shows a schematic frontal view of an analyte sensor system (1) according to another embodiment of the invention. This embodiment differs from the one described for Figure 2 only in the following aspect: the lower side of the housing comprises a **hole (413) which defines temperature** detection **area of the skin** (**414**) configured such that the temperature of the skin surface below the hole can be detecteded directly by the noncontact temperature sensor (7), in particular without obstruction by any physical object located between the noncontact temperature sensor and the skin (5) exposed by the hole (413). Preferably, the plaster (6) is also removed in this area so as to expose the skin (5) surface to the noncontact temperature sensor (7). To prevent that dirt, humidity or body liquids reach the circuit board or other parts of the interior of the housing a detection cell (10) is provided which comprises the noncontact temperature sensor (7), the lower side of the housing comprising the hole, and separating means (415), preferably separating walls, which seal off the detection cell (10) from the remaining interior space of the housing (4). The bottom- view of the body wearable analyte sensor device (2) indicated by A-A is depicted in Figure 5.

**Figure 5** shows a schematic bottom-up along with a frontal view of an analyte sensor system (1) according to the embodiment of the invention shown in Figure 4. In addition to the frontal view the figure also shows the bottom-up view schematic (upper part of the Figure marked A - A). The measurment cell (10) is separated from the remainder of the interior housing by separating means (415) such as a wall which is depicted in a circular shape but which of course could also take any other shape such as a square shape. Preferably, the separating means (415) are are watertight and thus effectivily prevent an ingress of humidity, dirt and fluid into the remaining space of the interior housing sealed off by the separating means (415).

## Example - Correction function for determination of glucose concentration based on the noncontact sensor determined temperature and the glucose sensor signal

Artificial interstitial fluid (AIF) can be obtained from a manufacturer (e.g. Simulated interstitial fluid, BZ254, from biochemazone.com). Alternatively, AIF can be prepared: 2.5 mM CaCl2, 10mM Hepes, 3.5 mM KCl, 0.7 mM MgSO4, 123 mM NaCl, 1.5 mM NaH2PO4, 7.4 mM saccharose is mixed, and the solution is adjusted to pH 7.5. Milli-Q water (18.2 M$\Omega$ cm, Millipore, Bedford, MA, USA) (see e.g. "Minimally Invasive Glucose Monitoring Using a Highly Porous Gold Micro needles-Based Biosensor: Characterization and Application in Artificial Interstitial Fluid", Paolo Bollella et al. Catalysts 2019, 9(7), 580; https://doi.org/10.3390/catal9070580). The AIF is then spiked with glucose to yield a defined glucose concentration of e.g. of 100 mg/dl.

A simulated chitosan/agarose hydrogel skin model (see Bollella et al., supra) is embedded in the glucose spiked AIF and the concentration of the glucose in the hydrogel is determined to be 100 mg/dl (YD1). The skin model is placed into a petri dish. Next a body wearable transcutaneous glucose sensor device of the invention is placed onto a simulated chitosan/agarose hydrogel skin model embedded in the AIF such that the transcutaneous glucose sensor is placed within the hydrogel. Moreover, a thermometer is placed into the hydrogel measure the temperature (XI) of the hydrogel independently from the temperature (X) measured by the non-contact temperature sensor. The setup is placed in an temperature controlled incubator. The temperature of the incubator is set such that the temperature measured by the non-contact temperature sensor (X) is 5, 10, 15, 20, 25, 30, 35, and 45 °C. The temperature (X) is determined by the noncontact tempeature sensor of the body wearable transcutaneous glucose sensor device. For each determined temperature x the glucose concentration detected by the transcutaneous glucose sensor (YD2) is recorded and the correction value (YC) is calculated by subtracting the predefined glucose concentration of the hydrogel (YD1) from the glucose concentration YD2 detected by the glucose sensor system. Based on the value pairs X and YC a glucose concentration correction value function can be determined which using the values in table 1 below. The function is

$$YC = -0{,}0412X^2 - 0{,}2163X + 50{,}13$$

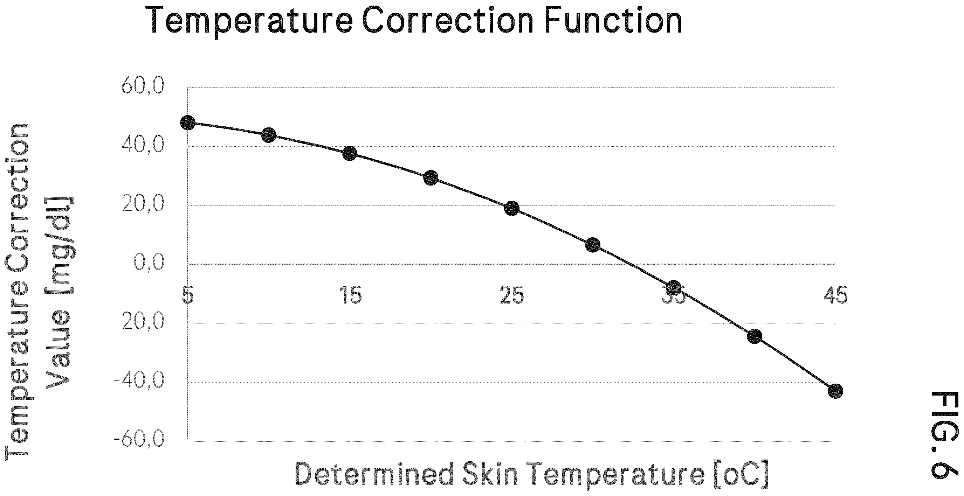

(see Figure 6).

This function can then be used by the glucose analyte sensor system of the invention to determining an analyte concentration based on a detected glucose sensor detection signal (YD2) and the determined temperature by the noncontact sensor (X). For example, if the glucose sensor detection signal indicates a concentration of YD2=143,8 mg/dl, based on the correction function a correction value of YC= 43,8 mg/dl is calculated and subtracted from YD2 to result in the determined glucose concentration of 100 mg/dl.

**Table 1**

| Comparison Measurement | Determined skin temperature (X) | Correction value (YC = YD2 - YD1) | Determined glucose concentration (YD2) | Set glucose concentration (YD1) |
|---|---|---|---|---|
| Nr. | [°C] | [mg/dl] | [mg/dl] | [mg/dl] |
| 1 | 5 | 48,0 | 148,0 | 100 |
| 2 | 10 | 43,8 | 143,8 | 100 |
| 3 | 15 | 37,6 | 137,6 | 100 |
| 4 | 20 | 29,3 | 129,3 | 100 |
| 5 | 25 | 19,0 | 119,0 | 100 |
| 6 | 30 | 6,6 | 106,6 | 100 |
| 7 | 35 | -7,9 | 92,1 | 100 |
| 8 | 40 | -24,4 | 75,6 | 100 |
| 9 | 45 | -43,0 | 57,0 | 100 |

In analogy to the above described glucose concentration correction value function, empirical determination of value pairs of the temperature (XI) of the hydrogel and the correspondingly determined temperature (X) determined by the non-contact temperature sensor at different temperatures of the hydrogel can be used to calculate a temperature correction value function which can be used to calibrate and correct for temperature measurement deviations by the noncontact temperature sensor vis-à-vis the thermometer based temperature measurement of the hydrogel.

It is clear to the skilled person that this methodology can also be applied to analyte sensor systems of the invention detecting analytes other than glucose.

**List of reference numbers**

1 analyte sensor system
2 body wearable analyte sensor device
3 transcutaneous analyte sensor
33 transcutaneous analyte sensor holder
331 separating walls
4 housing
41 lower side of housing
410 internal surface of the lower side of housing opposite of the surface facing the skin
411 lower side of housing with reduced thickness
412 temperature detection area of the lower side of housing
413 hole
414 temperature detection area of the skin
415 separating means
5 skin
6 plaster
7 noncontact temperature sensor
71 contact temperature sensor
72 glue
8 processor
81 memory

9       electronic unit
91      circuit board
10      detection cell
11      display device

**Claims**

1.  An analyte sensor system (1) which comprises

    • a body wearable analyte sensor device (2), comprising

        ◦ a transcutaneous analyte sensor (3),
        ◦ a housing (4) comprising a lower side (41) which can be attached to the skin (5) of a patient,
        ◦ a noncontact temperature sensor (7) which is adapted to detect the temperature of the lower side (41) of the housing) or to detect the temperature of the skin (5), and
        ◦ wherein the noncontact temperature sensor (7) faces without contact the lower side of the housing (41), and

    • an electronic unit (9) comprising a processor (8) configured to receive signals from the analyte sensor and from the noncontact temperature sensor (7).

2.  An analyte sensor system (1) according to claim 1, wherein the noncontact temperature sensor (7) is mounted on a circuit board (91), preferably on a printed circuit board.

3.  An analyte sensor system (1) according to claim 1 or 2, wherein the noncontact temperature sensor (7) is a passive infrared (PIR) sensor.

4.  An analyte sensor system (1) according to any of of claims 1 to 3, wherein housing further comprises a contact temperature sensor (71), optionally the contact temperature sensor is selected from the group consisting of a thermoelement, a thermistor, and a resistance temperature dectector.

5.  An analyte sensor system (1) according to claim 4, wherein contact temperature sensor (71) is attached to a part of the housing carrying the noncontact temperature sensor (7) or to a circuit board (91).

6.  An analyte sensor system (1) according to any of of claims 1 to 5, wherein the lower side (41) of the housing comprises a hole (413) defining a temperature detection area of the skin (414, 5) configured such that the temperature of the skin (5) below the hole (413) can be detected by the noncontact temperature sensor (7).

7.  An analyte sensor system (1) according to claim 6, wherein a detection cell (10) comprises the noncontact temperature sensor (7), the lower side (41) of the housing comprising the hole (413), and separating means (415) (prefeably separating walls) sealing off the detection cell (10) from the remaining interior space of the housing (4).

8.  An analyte sensor system (1) according to any of of claims 1 to 5, wherein the lower side (41) in a temperature detection area (41, 412) detected by the noncontact temperature sensor (7) has a reduced thickness relative to the thickness of other areas of the lower side (41).

9.  An analyte sensor system (1) according to any of of claims 1 to 8, wherein the analyte sensor system (1) further comprises a display device (11) configured to communicate with the body wearable analyte sensor device (2).

10. An analyte sensor system (1) according to any of of claims 1 to 9, wherein the transcutaneous analyte sensor (3) is a glucose sensor.

11. An analyte sensor system (1) according to any of of claims 1 to 10, wherein the processor (8) comprises a memory (81) with instructions which when executed cause the processor (8) to

    • receiving a temperature detection signal from the noncontact temperature sensor (7), and
    • determining the temperature of the lower side (41) of the housing or of the temperature of the skin (5) based on the received temperature detection signal from the noncontact temperature sensor (7).

**12.** An analyte sensor system (1) according to claim 11, wherein the memory (81) comprises further instructions which when executed cause the processor (8) to

- receiving an analyte sensor detection signal from the transcutaneous analyte sensor (3),
- determining an analyte concentration based on the analyte sensor detection signal and the determined temperature, and
- communicating the analyte concentration to the display device (11).

**13.** An analyte sensor system (1) according to claim 11 or 12, wherein the memory (81) comprises further instructions which when executed cause the processor (8) to

- comparing the determined temperature with a first and second predetermined reference temperature, and
- issueing a notification if the determined temperature is above the first predetermined reference temperature or below the second predetermined reference temperature, wherein the first predetermined reference temperature is higher than the second predetermined reference temperature.

**14.** According to another embodiment of the method of determing an analyte concentration using an analyte sensor system (1) according to any one of claims 1 to 13, the method comprising the steps of:

- receiving a temperature detection signal from the noncontact temperature sensor (7),
- determining the temperature of the lower side (41) of the housing (4) or of the temperature of the skin (5) a based on the received temperature detection signal from the noncontact temperature sensor (7),
- receiving an analyte sensor detection signal from the transcutaneous analyte sensor (3),
- determining an analyte concentration based on the analyte sensor detection signal and the determined temperature, and
- communicating the analyte concentration to the display device (11).

EP 4 201 326 A1

FIG. 1

FIG. 2

EP 4 201 326 A1

FIG. 3

EP 4 201 326 A1

FIG. 4

FIG. 5

A - A

Temperature Correction Function

FIG. 6

EP 4 201 326 A1

Europäisches Patentamt
European Patent Office
Office européen des brevets

# EUROPEAN SEARCH REPORT

Application Number

EP 21 21 6887

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/174638 A1 (KATSUKI KOJI [JP]) 21 July 2011 (2011-07-21) | 1,2,6-14 | INV. A61B5/145 A61B5/01 A61B5/00 |
| Y | * figures 9, 11 * * paragraphs [0093], [0106], [0109] – [0115], [0127], [0128], [0130], [0132], [0133] * | 3 | |
| X | US 2010/292557 A1 (PESACH BENNY [IL] ET AL) 18 November 2010 (2010-11-18) * figure 3a * * paragraphs [0104], [0110], [0134], [0136] – [0138] * | 1,4,5, 9-12,14 | |
| Y | US 2018/153454 A1 (HAYTER GARY A [US] ET AL) 7 June 2018 (2018-06-07) * paragraphs [0048], [0055], [0056], [0211], [0212] * | 3 | |
| A | US 2019/008424 A1 (SHIMIZU TAKESHI [JP]) 10 January 2019 (2019-01-10) * the whole document * | 1,14 | |
| A | US 10 201 295 B2 (VERILY LIFE SCIENCES LLC [US]) 12 February 2019 (2019-02-12) * the whole document * | 1,14 | TECHNICAL FIELDS SEARCHED (IPC) A61B |
| A | WO 2018/185138 A1 (ROCHE DIABETES CARE GMBH [DE]; HOFFMANN LA ROCHE [CH] ET AL.) 11 October 2018 (2018-10-11) * the whole document * | 1,14 | |

The present search report has been drawn up for all claims

| Place of search | Date of completion of the search | Examiner |
|---|---|---|
| Munich | 12 May 2022 | Trattner, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 6887

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2022

| Patent document cited in search report | | | Publication date | Patent family member(s) | | | Publication date |
|---|---|---|---|---|---|---|---|
| US 2011174638 | A1 | | 21-07-2011 | CN | 102178537 | A | 14-09-2011 |
| | | | | EP | 2345366 | A1 | 20-07-2011 |
| | | | | JP | 5462776 | B2 | 02-04-2014 |
| | | | | JP | 2011167503 | A | 01-09-2011 |
| | | | | US | 2011174638 | A1 | 21-07-2011 |
| | | | | US | 2015087941 | A1 | 26-03-2015 |
| US 2010292557 | A1 | | 18-11-2010 | EP | 2131723 | A2 | 16-12-2009 |
| | | | | ES | 2432404 | T3 | 03-12-2013 |
| | | | | US | 2010292557 | A1 | 18-11-2010 |
| | | | | WO | 2008114224 | A2 | 25-09-2008 |
| US 2018153454 | A1 | | 07-06-2018 | EP | 2720612 | A1 | 23-04-2014 |
| | | | | EP | 3556285 | A1 | 23-10-2019 |
| | | | | US | 2013158376 | A1 | 20-06-2013 |
| | | | | US | 2018153454 | A1 | 07-06-2018 |
| | | | | US | 2020029871 | A1 | 30-01-2020 |
| | | | | WO | 2012174563 | A1 | 20-12-2012 |
| US 2019008424 | A1 | | 10-01-2019 | CN | 109199408 | A | 15-01-2019 |
| | | | | EP | 3424412 | A1 | 09-01-2019 |
| | | | | US | 2019008424 | A1 | 10-01-2019 |
| US 10201295 | B2 | | 12-02-2019 | CN | 107405112 | A | 28-11-2017 |
| | | | | EP | 3244794 | A1 | 22-11-2017 |
| | | | | US | 2016262670 | A1 | 15-09-2016 |
| | | | | WO | 2016148906 | A1 | 22-09-2016 |
| WO 2018185138 | A1 | | 11-10-2018 | CA | 3053362 | A1 | 11-10-2018 |
| | | | | CN | 110461232 | A | 15-11-2019 |
| | | | | EP | 3606426 | A1 | 12-02-2020 |
| | | | | JP | 2020515353 | A | 28-05-2020 |
| | | | | JP | 2021180905 | A | 25-11-2021 |
| | | | | RU | 2745731 | C1 | 31-03-2021 |
| | | | | US | 2020029902 | A1 | 30-01-2020 |
| | | | | WO | 2018185138 | A1 | 11-10-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210259586 A1